# FASCICULE DE BREVET EUROPEEN

(11) **EP 1 914 535 B1**
(45) Date de publication et mention de la délivrance du brevet: **20.01.2010**
(21) Numéro de dépôt: 07117940.2
(22) Date de dépôt: 05.10.2007
(51) Int. Cl.: G01N 1/22, G01N 21/62

(54) **Caractérisation de gaz par spectrométrie optique à émission**
Gascharakterisierung für optische Emissionsspektrometrie
Characterisation of gas by optical emission spectrometry

(30) Priorité: 17.10.2006 FR 0654313
(43) Date de publication de la demande: 23.04.2008
(73) Titulaire: Alcatel Lucent, 75008 Paris (FR)
(72) Inventeur: Bounouar, Julien, 74000, ANNECY (FR); Godot, Erwan, 74000, ANNECY (FR); Thollot, Rémi, 74350, CRUSEILLES (FR); Brouillard, Adrien, 74000, ANNECY (FR)
(74) Mandataire: Korakis-Ménager, Sophie

(56) Documents cités:
- EP-A- 1 022 559
- US-A- 5 270 616
- US-A- 5 834 656
- US-A- 5 986 747
- US-B1- 7 123 361

## Description

La présente invention se rapporte à la caractérisation de gaz par spectrométrie: optique à émission (OES). Elle concerne plus particulièrement un dispositif de prélèvement en vue de l'analyse du gaz.

L'industrie des semi-conducteurs a un besoin réel de mieux connaître in-situ la composition des mélanges gazeux. On connaît des analyseurs permettant à ce jour d'obtenir une mesure qualitative et quantitative des espèces présentes dans un mélange gazeux, comme celui dont le principe est décrit dans le document EP-1 022 559 (US-6,643,014). Un tel analyseur comporte une source plasma qui excite un mélange de gaz présent dans une enceinte, En se désexcitant, les espèces gazeuses émettent des photons qui sont dirigés vers un spectromètre optique à émission par l'intermédiaire d'une fibre optique. Le spectromètre optique à émission (OES) sépare la lumière en longueurs d'ondes. La forme et l'intensité des raies du spectre de l'intensité lumineuse en fonction des longueurs d'onde permettent d'obtenir une information sur la nature des espèces présentes dans le gaz et leur quantité.
Toutefois dans le cadre d'une analyse quantitative des espèces gazeuses, cet analyseur n'est utilisable que si la pression reste constante. Si la pression totale est constante, l'analyseur permet de suivre l'évolution de pressions partielles de gaz. Lorsque la pression totale évolue, l'intensité totale du plasma évolue également en modifiant les proportions des pressions partielles. Deux paramètres font alors varier simultanément les proportions des espèces, ce qui rend toute interprétation impossible.

Par ailleurs le document US-5,986,747 décrit un autre dispositif d'analyse des gaz permettant de réaliser une mesure par spectrométrie optique à émission sur un mélange gazeux présent dans une chambre de procédé principale. Le dispositif comprend une chambre d'analyse de petite taille qui est reliée à la chambre de procédé. Le temps de résidence des gaz dans la chambre d'analyse est régulé par une vanne et un refoulement supplémentaire. Le système de refoulement supplémentaire peut maintenir la pression désirée dans la chambre d'analyse indépendamment de ce qui se passe dans la chambre de procédé.
Le procédé comprend le prélèvement d'un échantillon de gaz dans la chambre de procédé, l'isolation de cet échantillon dans la chambre d'analyse, l'excitation de l'échantillon dans la chambre d'analyse pour produire une radiation, et l'analyse de la radiation par un spectromètre.

Ce document concerne le prélèvement de gaz dans une chambre de procédé dans une plage de pression inférieure à 27 mbar. Il n'enseigne pas comment caractériser les espèces gazeuses sur une gamme de pression élevée allant jusqu'à la pression atmosphérique (1 bar) ou même au-delà,

La présente invention a pour but d'éliminer les inconvénients de l'art antérieur, en proposant un système d'analyse permettant d'associer les variations d'intensité du spectre seulement aux variations de pressions partielles, et non plus aux variations de puissance de la source plasma induites par des variations de la pression totale,

La présente invention a aussi comme but de permettre le fonctionnement de l'analyseur malgré une pression dans l'enceinte trop élevée pour obtenir un plasma, et ainsi de caractériser les espèces gazeuses d'un mélange sur toute la gamme de pressions allant de la pression atmosphérique (1 bar) ou de pressions supérieures, à des basses pressions inférieures à 10⁻² bar, même si cette pression varie dans le temps.

L'invention a encore pour but de proposer un procédé et un dispositif de prélèvement en vue de la détection d'espèces gazeuses dans une enceinte permettant de maintenir une pression constante au niveau de la source plasma, et ainsi s'affranchir de toute variation de pression dans l'enceinte.

L'invention a aussi pour but de proposer un procédé et un dispositif de prélèvement en vue de la détection d'espèces gazeuses dans une enceinte permettant une mesure en continu et en temps réel.

L'objet de la présente invention est un dispositif de prélèvement de gaz dans une enceinte en vue de son analyse communiquant d'une part avec une enceinte contenant le gaz à analyser reliée à un premier groupe de pompage, et d'autre part avec un analyseur comportant une chambre contenant un moyen d'excitation du gaz et un spectromètre optique à émission. Le dispositif inclut une portion de la canalisation reliant l'enceinte à l'analyseur, comprenant au moins une première vanne à conductance variable contrôlée en pression apte à imposer en aval une pression indépendante de la pression en amont à débit constant. La canalisation est reliée à un deuxième groupe de pompage permettant de maintenir un débit constant dans la portion de la canalisation incluse dans le dispositif.

Une vanne à conductance C variable est une vanne qui peut s'ouvrir ou se fermer partiellement de manière à obtenir une pression amont P1 différente de la pression aval P2 à débit Q constant, selon la relation : Q=C(P1-P2). Dans le cas présent, la pression aval P2 acceptable par l'analyseur est constante et inférieure à la pression amont P1. On voit que plus la pression amont P1 est élevée, c'est-à-dire plus la pression dans l'enceinte se rapproche de la pression atmosphérique, plus le rapport Q/C est grand, donc plus C doit être petite à débit Q constant. La conductance C sera d'autant plus petite que la vanne sera plus fermée.

Selon une forme d'exécution particulière de l'invention, le dispositif comprend au moins une vanne à conductance variable coopérant avec une vanne d'isolation. Une vanne d'isolation est une vanne qui peut être ouverte ou fermée, les pressions amont et aval étant égales.

Selon un mode de réalisation préféré de l'invention, la portion de canalisation est formée d'au moins deux branches parallèles comprenant chacune une vanne à conductance variable.

Avantageusement, la vanne à conductance variable est pilotée par un contrôleur de manière à maintenir une pression de consigne en aval.

L'invention a aussi pour objet un système d'analyse d'un gaz contenu dans une enceinte comprenant un analyseur, comportant une chambre contenant un moyen d'excitation du gaz et un spectromètre optique à émission, et un dispositif tel que décrit précédemment.

Avantageusement le système communique avec l'enceinte par l'intermédiaire du groupe de pompage de l'enceinte comprenant une pompe secondaire, le dispositif étant connecté au refoulement de la pompe secondaire. De façon alternative, lorsque l'enceinte est maintenue à basse pression au moyen d'un groupe de pompage comportant une pompe secondaire, telle qu'une pompe turbomoléculaire, le dispositif de prélèvement peut être placé au refoulement de la pompe secondaire. Au niveau du refoulement de la pompe secondaire, la pression dans la canalisation est plus élevée que dans l'enceinte et comprend sensiblement les mêmes proportions d'espèces gazeuses que l'on souhaite analyser. La différence de pression en amont et en aval de la vanne à conductance variable est alors suffisante pour conserver un débit constant et réaliser un prélèvement.

D'autres caractéristiques et avantages de la présente invention apparaîtront au cours de la description suivante d'un mode de réalisation, donné bien entendu à titre illustratif et non limitatif, et dans le dessin annexe sur lequel
- la figure 1 représente une installation comprenant un système d'analyse de gaz selon invention,
- la figure 2 montre un mode de réalisation du dispositif de prélèvement selon l'invention,
- la figure 3 montre un autre mode de réalisation du dispositif de prélèvement selon l'invention.

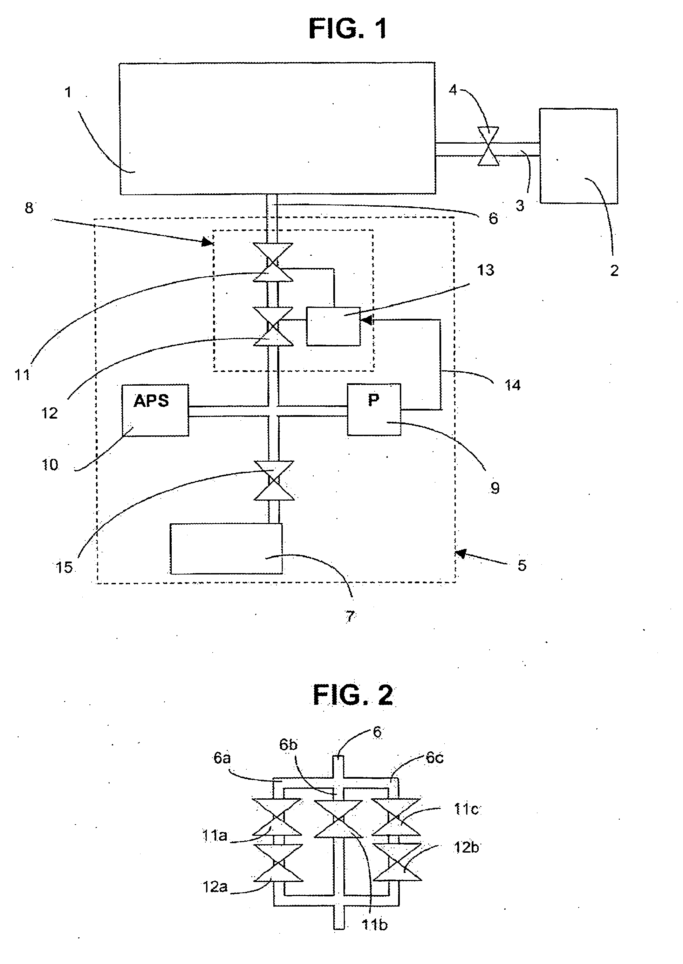

La figure 1 représente une enceinte **1** contenant le gaz à analyser reliée à un premier groupe de pompage **2** par une canalisation **3** munie d'une vanne **4**. L'enceinte **1** peut être par exemple une chambre de procédé, une chambre de transfert (load lock), ou un sas d'entrée / sortie. Dans l'enceinte **1**, la pression peut varier depuis la pression atmosphérique jusqu'à des basses pressions de l'ordre de 10⁻⁶ bar.

L'enceinte **1** est aussi reliée à un système d'analyse de gaz **5** selon l'invention. Ce système **5** communique avec l'enceinte **1** par une canalisation **6** connectée à un second groupe de pompage **7**. Le système **5** comprend un dispositif de prélèvement **8**. Un capteur de pression **9** et un analyseur **10** de référence «APS» de la société « ALCATEL », comprenant une chambre d'excitation et un spectromètre optique à émission (OES), sont raccordés sur la canalisation **6** en aval du dispositif de prélèvement **8**.

Le groupe de pompage **7** comporte avantageusement des moyens de vide secondaire de manière à obtenir une pression basse dans le système **5** qui procure un vide propre, évitant les pollutions par le dégazage des parois et la rétrodiffusion des gaz du pompage primaire. Le groupe de pompage de référence « DRYTEL » proposé par la société « ALCATEL » répond à ces critères puisqu'il permet d'obtenir un vide moléculaire atteignant 5.10⁻² mbar pour un débit de 2.10⁻³ mbar.l.s⁻¹ à moindre coût et pour un faible encombrement, grâce à une pompe moléculaire dite MDP (de l'anglais « molecular drag pump »), En outre on choisit la canalisation **6** du système **5** avec le volume le plus petit possible afin d'éviter la perturbation de la mesure par le dégazage des parois ou par la dilution de l'échantillon de gaz à mesurer et afin d'obtenir un temps de réponse rapide.

Le dispositif de prélèvement **8** selon l'invention comporte au moins une vanne, comme par exemple une vanne d'isolation **11** et une vanne à conductance variable **12** pilotée par un contrôleur **13.** Si la vanne à conductance variable est capable de fermer complètement le passage, il n'est plus nécessaire de lui adjoindre une vanne d'isolation. La vanne **12** à conductance variable est disposée sur la canalisation 6 de telle sorte que la pression en aval de la vanne **12** soit régulée à une pression de consigne choisie et indépendante de la pression en amont de cette vanne **12.** A cet effet la sortie **14** du signal du capteur de pression **10** placé en aval de la vanne **12** est reliée au contrôleur **13.**

Selon une forme particulière d'exécution de l'invention, plusieurs vannes à conductances variables peuvent être disposées en parallèle de manière à mieux adapter la conductance en fonction de la pression dans l'enceinte **1**, et pour couvrir toute la gamme de pression comprise entre la pression atmosphérique et 10⁻² mbar. Dans le mode de réalisation préféré représenté sur la figure 2, la portion de la canalisation **6** traversant le dispositif de prélèvement **8** comprend trois branches **6a, 6b** et **6c** disposées en parallèle. Chacune des canalisations **6a, 6b, 6c** comporte une vanne d'isolation **11a, 11b** et **11c** respectivement. Les canalisations **6a** et **6c** comportent chacune en outre une vanne à conductance variable **12a** et **12b** respectivement.

Un contrôleur **13** gère les ouvertures et fermetures des vannes d'isolation **11** et contrôle l'ouverture/fermeture des conductances des vannes à conductance variable **12**. Le contrôleur **13** peut comporter un système de régulation classique tel qu'un correcteur PID (pour « Proportionnel Intégral Dérivé »).

L'invention vise à conserver une pression constante acceptable par l'analyseur **10** quelle que soit la pression à l'intérieur de l'enceinte **1**. Pour cela, le prélèvement est effectué à un faible débit (de 1.10⁻² à 3.10⁻¹ mbar.l.s⁻¹, et de préférence 5.10⁻² mbar.l.s⁻¹) grâce une vanne à conductance variable **12** contrôlée en pression. De cette manière, il est possible de conserver en entrée de l'analyseur **10** une pression suffisamment faible pour permettre d'amorcer le plasma servant à la réalisation de la mesure sur le gaz. Avantageusement la pression en aval de la vanne à conductance variable **12** est de l'ordre de 50 mbar. De la mesure de la concentration des espèces gazeuses dans la canalisation **6** en aval de la vanne à conductance variable **12**, on peut déduire les concentrations des espèces gazeuses dans l'enceinte **1**.

Le second groupe de pompage **7** permet d'évacuer le flux d'échantillon gazeux, mais il est nécessaire, lorsque l'enceinte **1** est à basse pression, de modifier sa capacité de pompage afin de maintenir un débit d'échantillonnage constant. En effet, à basse pression, la différence de pression en amont et en aval de la vanne à conductance variable **12** devient trop faible pour conserver un débit constant

Une restriction **15**, telle qu'une vanne à conductance variable ou une restriction fixe, est placée en amont du groupe de pompage **7** pour permettre de réguler la vitesse de pompage du groupe de pompage **7** en fonction de la pression dans le système d'analyse **5** de manière à maintenir constant le débit d'échantillonnage,

Lorsque la pression dans l'enceinte **1** est élevée, la conductance de la première vanne à conductance variable **12a** est régulée dans le sens de la fermeture, et la conductance de la seconde vanne à conductance variable **12b** est régulée dans le sens d'une plus grande ouverture.

Lorsque la pression dans l'enceinte diminue, la vanne à conductance variable **12** s'ouvre au maximum et la restriction **15** commence à se fermer.

Le diamètre d'orifice de la conductance de la première vanne à conductance variable **12a** est avantageusement inférieur à 1 mm pour un flux de gaz de 1.10⁻² mbar.l.s⁻¹ à 50 mbar.l.s⁻¹. Si une autre vanne à conductance variable **12b** est disposée en parallèle de la première vanne **12a**, alors le diamètre d'orifice peut avantageusement être de l'ordre de 2 à 8 mm, et de préférence de l'ordre de 5 mm pour un flux de 10 mbar.l.s⁻¹ à 2.10³ mbar.l.s⁻¹. La plage de valeurs de la conductance de la seconde vanne à conductance variable **12b** est plus étendue, et permet des débits de 2.10³ mbar.l.s⁻¹ avec des diamètres d'orifice de l'ordre de 7 mm. Les vannes de référence « 132 » commercialisées par la société « « CELERITY » conviennent à cet usage. Ces vannes possèdent un temps de réponse extrêmement court, ce qui permet de faire une mesure en temps réel Elles prélèvent un petit débit de gaz à mesurer, ce qui permet de réaliser l'échantillonnage. Et elles peuvent réguler des pressions de l'ordre de 1 à 100 mbar, ce qui correspond à la gamme de pression qui convient à l'analyseur « APS ».

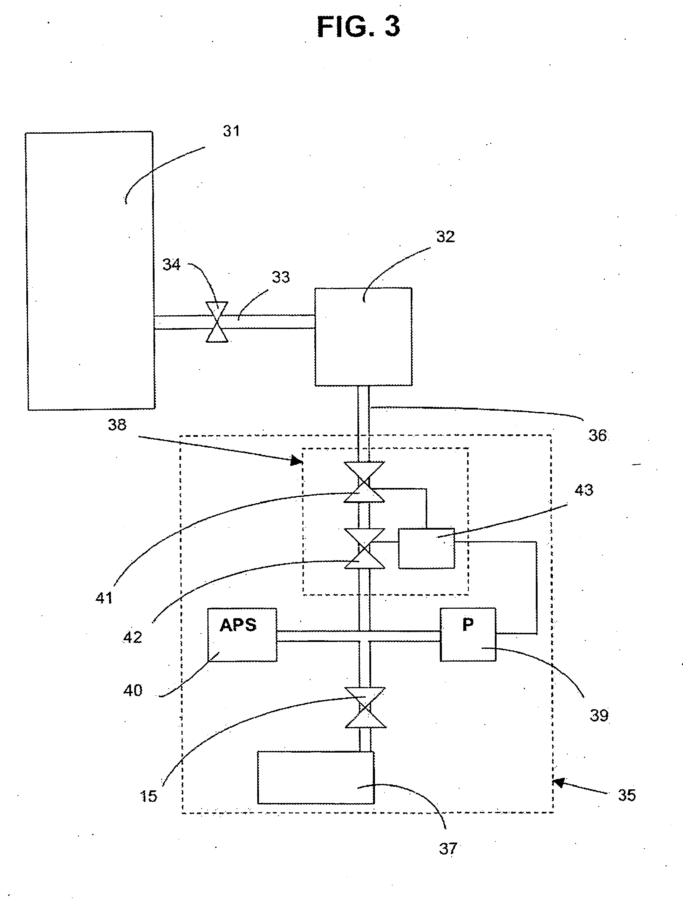

La figure 3 représente un autre mode de réalisation de l'invention dans lequel une enceinte **31** à basse pression est reliée à un premier groupe de pompage **32** par une canalisation **33** munie d'une vanne **34**. Le groupe de pompage **32** comporte une pompe secondaire telle qu'une pompe turbomoléculaire. Un système d'analyse de gaz **35** selon l'invention communique avec l'enceinte **31** au moyen d'une canalisation **36** connectée au refoulement de la pompe secondaire du groupe de pompage **32**.

Le système d'analyse de gaz **35** est relié à un second groupe de pompage **37**. Le système **35** comprend un dispositif de prélèvement **38**. Un capteur de pression **39** et un analyseur **40** sont raccordés sur la canalisation **36** en aval du dispositif de prélèvement **38**. Le dispositif de prélèvement **38** selon l'invention comporte au moins une vanne à conductance variable disposée sur la canalisation **36**, comme par exemple une vanne d'isolation **41** et une vanne à conductance variable **42** pilotée par un contrôleur **43**.

## Revendications

1. Dispositif de prélèvement de gaz dans une enceinte en vue de son analyse communiquant d'une part avec une enceinte (1) contenant le gaz à analyser, reliée à un premier groupe de pompage (2), et d'autre part avec un analyseur (10) comportant une chambre contenant un moyen d'excitation du gaz et un spectromètre optique à émission, le dispositif (8) incluant une portion de la canalisation (6) reliant l'enceinte (1) à l'analyseur (10), comprenant au moins une première vanne, **caractérisé en ce que** ladite vanne (12) est à conductance variable contrôlée en pression apte à imposer en aval une pression indépendante de la pression en amont à débit constant, et **en ce que** ladite canalisation (6) est reliée à un deuxième groupe de pompage (7) permettant de maintenir un débit constant dans la portion de la canalisation (6)

2. Dispositif selon la revendication 1, dans lequel la vanne (12) à conductance variable coopère avec une vanne (11) d'isolation,

3. Dispositif selon l'une des revendications 1 et 2, dans lequel la portion de canalisation (6) est formée d'au moins deux branches (6a, 6c) parallèles comprenant chacune une vanne (12a, 12b) à conductance variable.

4. Dispositif selon l'une des revendications 1 à 3, dans lequel la vanne (12) à conductance variable est pilotée par un contrôleur (13) de manière à maintenir une pression de consigne en aval.

5. Système d'analyse d'un gaz contenu dans une enceinte comprenant un analyseur, comportant une chambre contenant un moyen d'excitation du gaz et un spectromètre optique à émission, et un dispositif selon l'une des revendications précédentes.

6. Système selon la revendication 5, communiquant avec l'enceinte par l'intermédiaire du groupe de pompage de l'enceinte qui comprend une pompe secondaire, le dispositif étant connecté au refoulement de la pompe secondaire.

## Claims

1. Sampling device for gas in a chamber with a view to its analysis, connecting firstly with a chamber (1) containing the gas to be analyzed, connected to a first pumping unit (2), and secondly with an analyzer (10) comprising a chamber containing a means of excitation of the gas and an optical emission spectrometer the device (8) including a portion of the conduit (6) connecting the chamber (1) to the analyzer (10), comprising at least one first valve, **characterized in that** said valve (12) has pressure-controlled variable conductance able to impose downstream a pressure independent of the pressure upstream at a constant flow, and **in that** said conduit (6) is connected to a second pumping unit (7) allowing a constant flow to be maintained in the portion of the conduit (6).

2. Device according to claim 1, in which the variable conductance valve (12) works together with an isolation valve (11).

3. Device according to one of claims 1 and 2, in which the portion of conduit (6) is made up of at least two parallel branches (6a, 6c), each comprising a variable conductance valve (12a, 12b).

4. Device according to one of claims 1 to 3. in which the variable conductance valve (12) is controlled by a controller (13) so as to maintain a downstream setpoint pressure.

5. Analysis system for a gas contained in a chamber including an analyzer, comprising a chamber containing a means of excitation of the gas and an optical emission spectrometer, and a device according to one of the previous claims.

6. System according to claim 5, connecting with the chamber by means of the pumping unit of the chamber which includes a secondary pump, the device being connected to the discharge of the secondary pump.

## Patentansprüche

1. Vorrichtung zur Entnahme von in einem Behälter eingeschlossenen Gas zum Zweck der Analyse, welche einerseits mit einem das zu analysierende Gas einschließenden und an einen ersten Pumpstand (2) angeschlossenen Behälter (1), und andererseits mit einem Analysator (10), welcher eine ein Gaserregungsmiltel und ein optisches Emissionsspektrometer enthaltend Kammer umfasst, kommuniziert, wobei die Vorrichtung (8) einen Abschnitt der Rohrleitung (6), welche den Behälter (1) mit dem Analysator (10) verbindet, umfasst, mit mindestens einem ersten Ventil, **dadurch gekennzeichnet, dass** das besagte Ventil (12) einen variablen druckgesteuerten Leitwert aufweist, um am Ausgang einen von dem Eingangsdruck unabhängigen Druck mit konstanten Durchfluss aufzuerlegen, und dass die besagte Rohrleitung (6) an einen zweiten Pumpstand (7) angeschlossen ist, welcher es ermöglicht, einen konstanten Durchfluss in dem Abschnitt der Bohrleitung (6) aufrechtzuerhalten.

2. Vorrichtung nach Anspruch 1, wobei das Ventil (12) mit variablem Leitwert mit einem isolationsventil (11) zusammenwirkt.

3. Vorrichtung nach einem der Ansprüche 1 und 2, wobei der Abschnitt der Rohrleitung (6) aus mindestens zwei parallelen Verzweigungen (6a, 6c) mit jeweils einem Ventil (12a, 12b) mit variablem Leitwert besteht

4. Vorrichtung nach einem der Ansprüche 1 bis 3, wobei das Ventil (12) mit variablem Leitwert von einem Controller (13) gesteuert wird, um einen AusgangssoHdruck aufrecht zu erhalten,

5. System zur Analyse eines in einem Behälter eingeschlossenen Gases, umfassend einen Analysator mit einer Kammer, welche das Gaserregungsmittel und einen optischen Emissionsspektrometer enthält, und einer Vorrichtung nach einem der vorstehenden Ansprüche,

6. System nach Anspruch 5, welches über den eine Sekundärpumpe umfassenden Pumpstand des Behälters mit dem Behälter kommuniziert, wobei die Vorrichtung an die Druckseite der Sekundärpumpe angeschlossen ist.
